# EUROPEAN PATENT APPLICATION

(11) **EP 4 484 441 A1**
(43) Date of publication of application: **01.01.2025**
(21) Application number: 23181571.3
(22) Date of filing: 26.06.2023
(51) Int. Cl.: C07K 14/47, A61K 38/00, C07K 14/705, C07K 14/74, C07K 19/00

(54) **MHC-CLASS I DERIVED POLYPEPTIDE**

(71) Applicant: Eberhard-Karls-Universität Tübingen, 72076 Tübingen (DE)
(72) Inventor: Dennehy, Kevin, 72076 Tübingen (DE)
(74) Representative: Witte, Weller & Partner Patentanwälte mbB

(57) **Abstract**

The present invention relates to a polypeptide configured to bind a capture molecule when bound to an oligopeptide, a multimer comprising said polypeptide, a kit comprising the components of such multimer, a polynucleotide encoding such polypeptide, a host cell comprising such polypeptide, as well as methods involving the production and use of said polypeptide.

## Description

The present invention relates to a polypeptide configured to bind a capture molecule when bound to an oligopeptide, a multimer comprising said polypeptide, a kit comprising the components of such multimer, a polynucleotide encoding such polypeptide, a host cell comprising such polypeptide, as well as methods involving the production and use of said polypeptide.

### FIELD OF THE INVENTION

The present invention relates to the field of immunology, especially to the binding of MHC-derived polypeptides to capture molecules when bound to an oligopeptide, more specifically to the targeting of T cells by multimers of MHC-derived polypeptides.

### BACKGROUND

The main-histocompatibility-complex (MHC) is a cluster of genes in vertebrates encoding polymorphic polypeptides of MHC-class I. MHC-class I molecules (further referred to as MHC molecule) are located to the cell membrane and become bound to an oligopeptide, which can be pathogen-derived, to present it to the surrounding of the cell. MHC-class I molecules predominantly present oligopeptides of intracellular origin and comprise two polypeptides, i.e., a membrane-anchored MHC-class I α-chain and a β-2-microglobulin.

The combination from MHC molecule and bound oligopeptide (herein the oligopeptide-bound MHC molecule or MHC-derived polypeptide is also referred to as being "loaded") can be bound by a T-cell-receptor (TCR). TCR are proteins comprising highly diverse regions, which regions define the so-called "specificity" of a TCR. A TCR can highly specifically bind to one or few combinations of MHC molecule and oligopeptide, which oligopeptide is the so-called specific peptide of the respective TCR. Accordingly, each loaded MHC molecule can be attributed one or few TCR as binding partner, its so-called specific TCR. The binding between specific TCR and loaded MHC molecule depends on the binding of the specific peptide to the MHC molecule. In vertebrates such binding can have large influence onto the immune response as for example raising and/or modulating an immune response against a pathogen or disease or killing of the cell comprising the loaded polypeptide.

In the past years, production of soluble, loaded MHC molecules gained interest for targeting TCR or cells expressing a TCR based on the TCR's specificity. Knowing the specificity of a TCR or knowing which TCR binds a loaded MHC molecule is of great interest in medicine and research for being able to modulate the immune response in a desired way, by means to act against a disease. Soluble MHC molecules allow research of pairs of specific TCR and specific oligopeptide as well as application of such knowledge for diagnostic or therapeutic purposes. Also, independent from any medical applications, the specific nature of the interaction allows selective targeting of either a TCR by a loaded MHC molecule or vice versa for almost unlimited purposes in basic research and biotechnology.

Producing soluble, loaded MHC molecules imposes several challenges. MHC molecules are intrinsically instable if not loaded and thus become degraded by a producing cell or build inclusion bodies. Moreover, unloaded MHC molecules are oligopeptide-unreceptive, if not kept actively oligopeptide-receptive, as it is in vertebrate cells. They comprise two polypeptides and exhibit a low-affinity binding to their specific TCR, while high-affinity binding is desired for most applications. High-affinity binding, however, may in turn create disadvantages, in case a releasable binding is required. Furthermore, in research and medicine it is often required to maintain the functionality of the target, here the specific TCR.

WO2002054065 discloses a soluble MHC derived molecule comprising a strep-tag II binding to strep-tactin for multimerization. A multimerization enhances the affinity towards a capture molecule. The known MHC-derived molecule is produced as two distinct polypeptides by bacteria. However, production by bacteria yields the polypeptides as inclusion bodies. Thus, the production is time-consuming and expensive since it requires purification of the inclusion bodies as well as a refolding in the presence of a desired, appropriately restricted oligopeptide and another purification of the polypeptide.

WO2013030620 discloses a soluble MHC-class I derived molecule which can be expressed as a fusion protein, wherein the region of the MHC-class I molecule binding the oligopeptide is kept permanently in a peptide-receptive conformation. Said known MHC-class I derived molecule comprises a tag to be biotinylated. The biotin residue can be bound to streptavidin for multimerization. However, such polypeptide requires refolding in the presence of a desired, appropriately restricted oligopeptide and an additional biotinylation step, which is time-consuming, makes further purification of the biotinylated product necessary, and raises the costs for the production.

Thus, it is an object of the present invention to provide an improved MHC-derived polypeptide with which the disadvantages of the prior art are eliminated or at least mitigated. Especially, such an MHC-derived polypeptide should be provided which is fast, cheap and easy to produce and to multimerize. This object is fully solved by the present invention.

### SUMMARY OF THE INVENTION

In an aspect of the invention, the above identified disadvantages and others are overcome by providing a polypeptide comprising a single continuous amino acid chain, said amino acid chain comprising a first domain derived from β-2-microglobulin, a second domain derived from an MHC-class I α-chain, and a multimerization-tag, wherein said second domain comprises a first region configured to bind an oligopeptide, said first region comprising two α-helices being connected via a disulphide bond, a second region configured to bind a capture molecule when the first region is oligopeptide-bound; and said multimerization-tag being configured to bind a streptavidin-derived polyvalent agent.

Throughout herein the term "domain" is used to define a portion of the MHC-derived polypeptide, to which certain functions may be attributed throughout this specification. The term may relate to the primary, secondary, or tertiary structure of the polypeptide according to the invention.

A domain or a polypeptide being "derived" from a natural polypeptide is to be understood as comprising fragments of the natural polypeptide and fulfilling the main functionalities of the natural protein. With respect to an MHC-class I α-chain, a main functionality is the binding of an oligopeptide such that binding to one or few specific TCR is allowed. With respect to the β-2-microglobulin, a main functionality is having the property to stabilize the correct folding of the MHC-class-I α-chain and thus the correct and stable folding of the whole MHC-class I molecule. A domain or a polypeptide being "derived" from a natural polypeptide may also comprise modifications, deletions or insertions that do not interfere negatively with those main functionalities.

The oligopeptide binding region of an MHC molecule, which is equivalent to the first region of the second domain, comprises two α-helices forming a groove, in which the oligopeptide is bound, said oligopeptide usually having a length of about 8 to 11 amino acids. A link between the two α-helices of the first region of the second domain keeps said first region oligopeptide-receptive, which means the first region does not lose its ability to bind an oligopeptide when no oligopeptide is bound. Thus, a disulphide bond linking said α-helices makes a time-consuming refolding in the presence of a desired, appropriately restricted oligopeptide or fragments thereof obsolete. Advantageously, this facilitates the production of the polypeptide technically and shortens the production-time. Furthermore, it allows individual loading of the ready-to-use sold polypeptide by the user with a desired oligopeptide or mixture of polypeptides.

Choosing a disulphide bond being the linkage between the two α-helices is advantageous since disulphide bonds do naturally occur in proteins, especially also in MHC molecules. Thus, this linkage is easily to be introduced during the production step of the polypeptide.

Herein the term "multimerization-tag" is to be understood as a domain of a polypeptide comprising a region configured for direct and immediate binding of a polyvalent agent without an intermediary structure or residue. A multimerization-residue in contrast, has to be bound to a tag configured to be modified accordingly and this multimerization residue in turn could bind a polyvalent agent.

The multimerization-tag is configured to bind a streptavidin-derived polyvalent agent. This binding advantageously allows multimerization of the polypeptide to build a stable multimer, since streptavidin or derivatives developed in the past years and their ligands as biotin, strep tag, or strep tag II are known to have a remarkably low dissociation constant and establish a binding known to be of high affinity. Multimerization of the polypeptides advantageously enhances the avidity of said multimer towards the capture molecule over the sum of the affinities of each individual polypeptide.

Direct immediate binding between multimerization-tag and polyvalent agent advantageously allows to reduce the complexity of the polypeptide itself, since an element (residue or the like) is waived, the complexity of the polypeptides production, since any step for introducing said intermediary structure is obsolete, and the complexity of the polypeptide's binding to a polyvalent agent, since not two binding sites (tag to multimerization-residue and multimerization-residue to polyvalent agent) but only one (multimerization-tag to polyvalent agent) needs to be controlled. Furthermore, such multimerization-tag may also be used for purification of the polypeptide without further modification, for example by affinity-chromatography.

In an embodiment, the polypeptide is produced by a eukaryotic cell, preferably by a mammal cell, most preferably by a Chinese hamster ovary (CHO) cell, a J558L cell, a WEHI-231 cell, or a human cell.

Production of the polypeptide from a eukaryotic cell advantageously allows to avoid the necessity for an unfolding and refolding step of the polypeptide in the presence of peptide antigen or fragments thereof from inclusion bodies.

In an embodiment of the present invention, the multimerization-tag is configured to bind reversibly to a streptavidin-derived polyvalent agent, such that said binding can be released by elution at 4°C.

Reversibility of the binding between multimerization-tag and polyvalent agent advantageously allows to release binding between the polypeptide and the capture molecule by disassembling the multimer. This is especially advantageous for further processing of an unknown capture molecule, or a polypeptide loaded with an unknown oligopeptide, for example, for its identification, characterization, or investigation.

Throughout this specification, binding between biotin and a streptavidin derived polyvalent agent, is not considered to be reversible, since to release the binding may influence a not neglectable fraction of the target negatively, especially when the target is a capture molecule bound to a cell.

Reversibility can be achieved by introducing a strep-tag II peptide, for example. This advantageously allows direct immediate binding between multimerization-tag and streptavidin-derived polyvalent agent, but is still releasable under relatively mild conditions, such as elution at 4 °C.

In contrast, the multimer disclosed in WO2013030620 is not easily to be removed for reestablishing or protecting the functionality of the TCR and/or a T cell expressing it, since harsh conditions including heating are required for this purpose, which affects a bound cell and/or TCR negatively.

In another embodiment, the multimerization-tag comprises at least two identical or different sections, wherein each of said sections is configured to bind a streptavidin-derived polyvalent agent.

The at least two sections configured to bind a streptavidin-derived polyvalent agent may be connected via an amino acid spacer having a length of up to 15 amino acids. Alternatively, the sections may be located directly adjacent to each other with respect to the primary structure of the polypeptide.

The stability of the multimer resulting from the known polypeptide disclosed in WO2002054065 is limited due to the low affinity between strep-tag or strep-tag II and streptavidin or a streptavidin derived polyvalent agent as compared to streptavidin and biotin. In contrast, providing at least two regions configured to bind the streptavidin-derived polyvalent agent, advantageously improves the avidity of the binding between multimerization-tag and polyvalent agent. Without being bound to the theory, this is suggested to be due to enhanced probability of an encountering of a region configured to bind the polyvalent agent and one of the corresponding binding sites of the polyvalent agent. Thus, the stability of a multimer is enhanced such that its production becomes more efficient and thus easier, and its use is facilitated since the reaction-conditions can be less controlled.

More advantageously, such multimerization-tag according to the invention also allows a facile disassembly of a multimer since release of the binding between multimerization-tag and polyvalent agent can be easily achieved by elution with a substance competing with the multimerization-tag for the binding to an individual binding site of the polyvalent agent. Harsh conditions such as heating are avoided.

In a further embodiment, the multimerization-tag is a twin-strep-tag.

A twin-strep-tag comprises two identical regions configured to bind a streptavidin-derived polyvalent agent, which regions are connected via an amino acid linker having a length of up to 15 amino acids.

A twin-strep-tag, known to the skilled person, for example, from WO02077018A1 in the context of protein-analytics or protein-purification, advantageously allows to easily disassemble a multimer since the respective binding between multimerization tag and polyvalent agent is reversible. More advantageously, such multimerization-tag allows a notably facile disassembly of said multimer since release of the binding between multimerization-tag and polyvalent agent can be easily achieved by elution with, for example, biotin, a peptide comprising a strep-tag or a peptide comprising a strep-tag II.

In another embodiment, the twin-strep-tag comprises the amino acid sequence of SEQ ID NO: 1.

Use of a twin-strep-tag comprising such sequence has allowed reliable and reversible binding of the multimerization-tag to a streptavidin-derived polyvalent agent.

In another aspect of the present invention, the multimerization-tag is linked to the C-terminal end of the second domain via a first amino acid linker, wherein preferably the first amino acid linker has a length of 4, 5, or 6 amino acids.

An amino acid linker advantageously spatially separates the second domain of the polypeptide and the multimerization-tag. This ensures that both may fold correctly without being sterically hindered by secondary or tertiary protein structures of each other imposing structural influence along the amino acid chain or via back-folding, respectively.

In this embodiment, the length of 4, 5, or 6 amino acids ensures sufficient spatial separation while not introducing an unnecessarily long peptide, which may counteract the avidity-enhancing effect of a multimerization of the polypeptide.

In another embodiment, the first amino acid linker most preferably comprises the amino acid sequence of SEQ ID NO: 2.

This sequence is highly flexible due to a high content of the small amino acid glycine. The flexibility advantageously supports the binding between the multimerization-tag and the polyvalent agent by giving the multimerization-tag a certain degree of freedom to orient such as to encounter the corresponding binding site on the polyvalent agent. Furthermore, the hydrophilic serine-residue ensures sufficient solubility and thus stability.

In another aspect of the present invention, the C-terminal end of the first domain is linked to the N-terminal end of the second domain via a second amino acid linker, wherein preferably the second amino acid linker has a length of 12 to 18 amino acids.

In this embodiment, the length of 12 to 18 amino acids ensures sufficient spatial separation of the first and second domain as well as spanning of the distance between the C-terminus of the first domain and the N-terminus of the second domain. Said distance is predetermined due to the quaternary structure of an MHC molecule. At the same time, it is avoided to introduce an unnecessarily long peptide, which may counteract the correct interaction of MHC-class I derived α-chain and β-2-microglobulin.

In another embodiment, the second amino acid linker most preferably comprises the amino acid sequence of SEQ ID NO: 3.

This sequence is highly flexible due to a high content of the small amino acid glycine. The flexibility advantageously supports the binding between the first and the second domain by giving both a certain degree of freedom to orient such as to position correctly with respect to the quaternary structure of an MHC molecule. Furthermore, the hydrophilic serine-residue ensures sufficient solubility and thus stability.

In another aspect of the invention, the capture molecule comprises a T-cell-receptor (TCR) or a TCR-like chimeric antigen receptor (TCR-like CAR) or a fragment thereof.

T-cell-receptors are natural ligands of loaded MHC molecules consisting of two amino acid chains forming a protein complex. TCR-like chimeric antigen receptors (TCR-like CAR) are recombinant engineered molecules comprising all fragments of a TCR mediating the binding between TCR and MHC molecule and conferring the specificity towards MHC molecule and oligopeptide to a TCR. Thus, it is advantageous to choose the capture molecule to be a TCR, a TCR-like CAR, or a fragment thereof, since this way the need to provide an entirely new type of artificial capture molecule is circumvented.

The capture molecule can be entirely or partially synthesized artificially, produced artificially by a cell or a cell-free lysate, isolated artificially from a cell naturally producing it, or produced naturally by a cell. The capture molecule can be a single molecule or a group of molecules, such as a protein complex. The capture molecule is part of a target and can be soluble, secreted, or bound to or associated with one or more other structures. For example, it can be bound to a carrier or membrane-anchored.

The capture protein being bound with another structure allows to target not only the capture molecule itself, but also the other structure.

The term "target" refers to the capture molecule and may further include another structure bound to the capture molecule as, for example, a cell.

The verb "to target" includes to induce binding of the polypeptide to the capture molecule. Such binding can have consequences as, for example, making the target distinguishable from non-targets, which can finally allow its identification, and/or influencing the behavior or state of a cellular target, which consequences are meant to be included in the verb "to target". If a capture molecule is targeted, simultaneously another structure bound to the capture molecule is meant to be targeted, too.

The carrier can be, for example, the bottom of a tube, for example plastic, or a material suitable for use as a stationary phase of a chromatographic column or capillary, for example a gel preferably made from dextran or agarose, or a material from which beads or magnetic beads can be made, which are well known in the art. This advantageously allows to separate polypeptides loaded with the specific oligopeptide of the respective TCR or TCR-fragment from an oligopeptide-library loaded to polypeptides according to the invention by separation-methods as chromatography, magnetic-bead separation, pull-down assay, or assays, wherein the loaded polypeptide becomes immobilized to the bottom of a tube and can be released later after having washed away all non-immobilized loaded polypeptides.

The membrane can be an artificial or natural layer, for example a lipid-mono- or bi-layer, wherein the membrane is embodied preferably spherical with a sphere having a diameter between 10 nm and 1 mm, preferably between 50 nm and 250 µm. The spherical membrane may comprise a compartment inside it, which may contain targets of interest.

Spherical membranes of such size advantageously allow to target via the binding of the capture molecule by the loaded polypeptide the spherical membrane and/or what might be contained inside the sphere. Preferably, the membrane is a cell-membrane or part of a lipid nanoparticle, most preferably a eukaryotic cell-membrane, further preferably a T cell, highest preferred a CD8⁺ T cell. Advantageously, CD8⁺ T cells express TCR being the natural ligands, i.e., capture molecules, of MHC-class I molecules.

In another embodiment of the present invention, the first domain is derived from a human β-2-microblobuline and preferably comprises the amino acid sequence of SEQ ID NO: 4.

The use of a human β-2-microglobulin advantageously allows to enhance the compatibility of the polypeptide for binding to human capture molecules as well as for application in conjunction with human samples or to the human body.

In another embodiment of the present invention, the second domain derived from an MHC-class I α-chain comprises a modification reducing the binding to CD8.

Such modification advantageously allows to make the kinetics of the binding between polypeptide and specific TCR less controlled by interactions with CD8 and more dependent on the loaded polypeptide-to-TCR interaction itself. Further advantageously, it reduces unspecific binding of the polypeptide in the presence of CD8. Thus, the targeting becomes more accurate.

In another embodiment of the present invention, the MHC-class I α-chain is a human leukocyte antigen (HLA).

The use of a human MHC-class I α-chain, a so-called HLA, advantageously allows to further enhance the compatibility of the polypeptide for binding to human capture molecules as well as for application in conjunction with human samples or to the human body.

In another embodiment of the present invention, the MHC-class I α-chain is HLA-A*01.

MHC-class I α-chain of allotype HLA-A*01 is advantageous in that the HLA-A*01 allotype is highly prevalent. Therefore, the fraction of TCRs binding to combinations from an HLA-A*01-derived polypeptide and a bound oligopeptide is high. Thus, polypeptides or respective multimerized polypeptides comprising a second domain derived from HLA-A*01 are to be used for a major fraction of patients or, generally speaking, in most cases of application.

In another embodiment, the amino acid sequence of the polypeptide comprises the amino acid sequence of SEQ ID NO: 5.

The amino acid sequence of SEQ ID NO: 5 comprises from N-terminus to C-terminus in this order: a human β-2-microglobulin sequence, a second linker, an HLA-A*01-derived sequence (which is an MHC-class I α-chain), a first linker, and a twin-strep-tag.

The two cysteines building the disulphide bond linking the two α-helices of the first region of the second domain of the polypeptide are located at positions 83 and 139 of the HLA-A*01 sequence. Such positioning has shown to keep the first region of the second domain peptide-receptive while not interfering with the binding between polypeptide and capture molecule.

In another aspect of the invention, the polypeptide is suitable for use in staining, visualization, localization, quantification or separation of cells or capture molecules, for use in stimulation, activation, or sorting of cells, for use in techniques comprising the generation or use of an oligopeptide-library, or for use in diagnosis or therapy of a disease.

Throughout herein, "staining" involves the binding of loaded polypeptide and specific TCR to associate signal residues in close proximity to the target such that by detection of the signal residue the presence and/or localization of the target can be unraveled.

Throughout herein, "visualization" means making the presence of a target visible for a human eye or detectable under a microscope or by a cell sorting machine.

Throughout herein, "localization" means localizing the target on a cell's surface or on a type or population of cells in the context of a tissue, for example by microscopy.

Throughout herein, "quantification" means using information gained from staining, visualization or localization of cells or capture molecules for calculating an absolute or relative amount of target present in the respective sample.

Throughout herein, "separation" involves using information gained from staining, visualization or localization of cells or capture molecules for isolating targets from a respective sample.

Throughout herein, "stimulation" and "activation" are used interchangeably and involve binding of the polypeptide to a cell-membrane-anchored capture molecule for influencing the behavior of a cell, for example, with respect to proliferation, gene expression, or production of proteins.

Throughout herein, "sorting of cells" involves using information gained from staining or visualization of cells for isolating said cells from a sample.

Such uses do comprise targeting a capture molecule and allow investigation of pairs of capture molecule and loaded polypeptide. They advantageously allow to target capture molecules not only based on the structure of the capture molecule but based on its ability to bind a combination from specific oligopeptide and MHC-derived polypeptide, which raises the targeting from a structure-dependent to a functionality-dependent level.

Use of the polypeptide in diagnosis or therapy of a disease is advantageous in that it facilitates them and allows individualized medicine.

Regarding a therapy, it allows identification of specific TCRs against an individual oligopeptide, which is an antigen associated with an individual disease as cancer. The identification process is fastened due to the fastened production of the polypeptide and its easy multimerization. Furthermore, cancer specific CD8⁺ T cells can be primed *in vitro* by use of such multimerized polypeptide and transferred later into a mammal suffering from cancer, after the release of the polypeptide from the cell, wherein said release advantageously does not damage the cell or its functionality.

Regarding diagnosis, it advantageously allows monitoring of CD8⁺ T cell responses for assessing efficiency of a therapeutic treatment involving transfer of CD8⁺ T cells or for identifying oligopeptides against which the CD8⁺ T cell response is directed. Also here, the methods become faster and easier by use of an oligopeptide according to the invention.

In embodiments, wherein the binding between multimerization-tag and polyvalent agent is reversible, the mentioned uses are further improved by allowing to disassemble a multimer comprising the polypeptide for stopping an influence onto the behavior and/or phenotype of the cell or for making the capture molecule or the cell available to uses hampered by the polypeptide bound to the capture molecule. For example, T cells targeted for expansion and subsequent transfer into a mammal for therapeutic purposes do not suffer losses or damages during release of the polypeptide.

In another embodiment of the present invention, the polypeptide comprises an oligopeptide bound to the first region of the second domain.

The polypeptide comprising an oligopeptide bound to the second domain advantageously ensures that the polypeptide can be used by a user without further loading step.

In another aspect of the invention, it is provided a kit for targeting a capture molecule, said kit comprising the following components: a polypeptide according to the invention and a streptavidin-derived polyvalent agent.

The kit can comprise polypeptides, wherein the second domain of all polypeptides is derived from a single allotype or from at least two different allotypes. This advantageously allows the user to choose a particular allotype of the polypeptide or to combine several.

The kit may further comprise oligopeptides, wherein the oligopeptides can be present in a mixture from identical or not identical oligopeptides. Identical oligopeptides allow to load polypeptides with a specific oligopeptide and thus to target a specific capture molecule. Not identical oligopeptides advantageously allow to load polypeptides with an oligopeptide-library and thus, for example, to screen the library for oligopeptides to be bound by a capture molecule of interest.

Another subject-matter of the invention is a polynucleotide encoding the polypeptide according to the invention.

Still another subject-matter of the invention is an expression vector comprising the polynucleotide according to the invention.

The expression vector advantageously can be introduced into a cell for production or expression of the polypeptide.

In one embodiment, the expression vector is selected from the group consisting of: a plasmid, a retrovirus, or an mRNA.

A plasmid, retrovirus, or mRNA is advantageous in that it allows expression by eukaryotic cells and allow use of standard methods well known in the art for production of the polypeptide. A retrovirus or mRNA is further advantageous since it allows expression in mammal cells, as for example human cells. Also, plasmids can be intermediary suitable for expression in mammals via introduction of the plasmid into a retroviral particle or directly by introduction into yeast. Expression, i.e., production, of the polypeptide by eukaryotic cells advantageously avoids the formation of inclusion bodies from the polypeptide due to misfolding of the polypeptide, as it is known for production by bacteria.

Another subject-matter of the invention is a host cell comprising the polynucleotide or the expression vector according to the invention.

In one embodiment, the host cell is selected from the group consisting of: a bacterial cell, a mammal cell, a yeast cell, or an insect cell, wherein preferably the cell is a mammal cell, most preferably a Chinese hamster ovary (CHO) cell, a J558L cell, a WEHI-231 cell or a human cell.

Another subject-matter of the invention is a multimer comprising at least one polypeptide according to the invention and a streptavidin-derived polyvalent agent bound to the at least one polypeptide via its multimerization-tag.

Multimers of MHC-derived polypeptides are known to have a higher avidity towards their capture molecules as compared to monomers or the sum of the affinity of the monomers building the multimer. Thus, a multimer advantageously has a higher avidity towards the capture molecule and allows reliable targeting of the capture molecule.

The multimer comprises at least 2 of the polypeptides. Preferably, the multimer is a tetramer. Tetramers of MHC-derived polypeptides are known to have a sufficient avidity towards their capture molecule.

The streptavidin-derived polyvalent agent can be, for example, multimers of streptavidin, a streptavidin-mutein, or multimers of a streptavidin-mutein. The streptavidin-muteins have been developed in the past years by modifying streptavidin to bind a strep-tag or a strep-tag II, respectively, with a higher affinity than natural streptavidin.

In one preferred embodiment, the polyvalent agent is strep-tactin or multimerized strep-tactin.

Strep-tactin is a streptavidin-derived polyvalent agent, a so-called streptavidin-mutein, known for its ability to bind certain peptide-tags, which are derived from the strep-tag, especially the strep-tag II.

In another embodiment, the polyvalent agent is covalently bound to at least one signal-residue, the signal-residue being preferably a fluorophore. The fluorophore can be a protein, or a protein complex being fused to the streptavidin-derived polyvalent agent, as for example a phycoerythrin (PE) derived fluorophore, green fluorescent protein (GFP) or enhanced GFP (eGFP).

A "signal-residue" is understood to be a residue suitable for emitting an identification sign or catalyzing a chemical reaction, which itself or whose products emit a signal or identification sign. The signal is preferably electromagnetic radiation. The signal-residue advantageously allows to detect the multimer and thus also a capture molecule bound to the polypeptide.

The signal residue being a fluorophore is advantageous since fluorophores are widely used and well known in the field as well as in the uses the multimer or the polypeptide is provided for.

Another subject-matter of the invention is a method for targeting a capture molecule comprising the steps of providing a polypeptide according to the invention; contacting a desired oligopeptide with the polypeptide to generate an oligopeptide-bound polypeptide; contacting the oligopeptide-bound polypeptide with the polyvalent agent to generate a multimer; and contacting a capture molecule with the multimer to obtain a targeted capture molecule.

In another embodiment, the polyvalent agent is covalently bound to at least one signal-residue, preferably to a fluorophore, and optionally the method for targeting a capture molecule comprising the step of: detecting a signal from the signal-residue.

This embodiment allows the method to be easily applied for uses the multimer or polypeptide is intended for, and which also require the polypeptide or multimer to be detected, since fluorophores are widely used and well known in the field.

Yet another subject-matter of the invention is an expression system comprising the expression vector and the host cell according to the invention or a cell-free lysate.

In one embodiment, the expression system is configured to produce the polypeptide and/or the multimer.

An expression system configured to produce the polypeptide advantageously allows a user to produce the polypeptide while the polyvalent agent and/or the oligopeptide may be bought externally, which facilitates the production process of the multimer.

An expression system configured to produce the multimer advantageously allows a user to produce the polypeptide and the polyvalent agent and optionally also the oligopeptide from a single cell culture, which allows to fully produce the multimer in-house without having to rely on bought products. Advantageously this combines the polypeptide production step and the multimerization step to a single multimer production step.

It is understood that the features mentioned above and those yet to be explained below can be used not only in the particular combination given, but also in other combinations or in isolation, without departing from the scope of the present invention. Especially, the features, characteristics, embodiments and advantages mentioned with respect to the polypeptide similarly apply to the multimer, the kit, the polynucleotide, and the host cell comprising, encoding, or expressing the polypeptide.

The invention is now described and explained in further detail by referring to the following non-limiting examples and drawings.

### BRIEF DESCRIPTION OF THE FIGURES

Fig. 1: Schematic view of a polypeptide according to the invention and an oligopeptide bound to the first region of the second domain.
Fig. 2: Schematic view of a multimer according to the invention, four oligopeptides, and a target bound to said multimer.
Fig. 3: Photo of a Coomassie-stained reducing SDS-PAGE analysis of the produced polypeptide 10. To the left lane 1 the polypeptide 10 was applied. To the right lane 2 medium was applied as negative control.
Fig. 4: FACS-plots depicting CD8⁺ Jurkat76 cell lines expressing the specific or a control TCR and stained with multimers according to the invention, which are either unloaded or loaded with an oligopeptide represented by SEQ ID NO: 6.

### EXAMPLES

### 1. Polypeptide of the invention

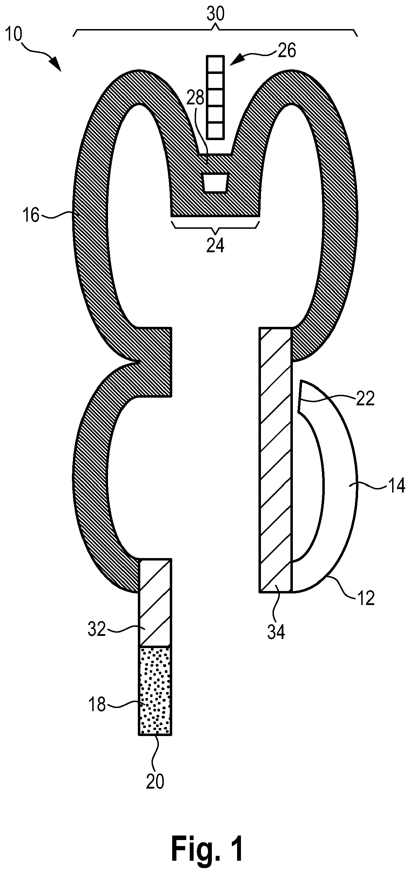

Fig. 1 depicts a schematic view of a polypeptide 10 according to the invention comprising a single continuous amino acid chain 12, said amino acid chain 12 comprising a first domain 14, a second domain 16, and a multimerization-tag 18.

The single continuous amino acid chain 12 comprises the amino acid sequence of SEQ ID NO: 5. The amino acid chain 12 has a C-terminal end 20, which is located at the multimerization-tag 18, and a N-terminal end 22, which is located at the first domain 14. The polypeptide 10 has a molecular weight of about 50kD.

The first domain 14 is derived from a human β-2-microglobulin and has the amino acid sequence of SEQ ID NO: 4.

In other embodiments, the first domain 14 can be derived from any specie's β-2-microglobulin, preferably from a mammal, for example from a mouse.

The second domain is derived from HLA-A*01, which is an MHC-class I α-chain. More specifically, the HLA-A*01 is a modified extracellular portion from HLA-A*01, wherein the alanine at position 245 was exchanged by a valine (so-called A245V mutation) to reduce binding to CD8 and two cysteines were added in the HLA sequence at positions 84 and 139 to allow formation of a disulfide bond 28. The second domain has the amino acid sequence of SEQ ID NO: 7.

In other embodiments, the second domain 16 can be derived from any specie's MHC-class I α-chain, preferably from a mammal, for example from a mouse.

The second domain 16 comprises a first region 24 configured to bind an oligopeptide 26, said first region 24 comprising two α-helices (not shown) being connected via the disulphide bond 28. The second domain 16 further comprises a second region 30, which is configured to bind a capture molecule (not shown) when the first region 24 is oligopeptide-bound. The first region 24 and the second region 30 can be distinct from each other or may overlap partially.

The oligopeptide 26 has a length of 10 amino acids and the amino acid sequence of SEQ ID NO: 6. In other embodiments, the oligopeptide can be any oligopeptide having a length of about 8 to 11 amino acids.

The multimerization-tag 18 is a twin-strep-tag having the amino acid sequence of SEQ ID NO: 1 and comprising two identical sections (not shown) being a strep-tag II having the amino acid sequence of SEQ ID NO: 8. A strep-tag II is configured for direct and immediate binding of a streptavidin-derived polyvalent agent (not shown).

In other embodiments, the multimerization-tag 18 can be any tag configured for direct and immediate binding of a streptavidin-derived polyvalent agent. For example, the multimerization-tag 18 can be a strep-tag, a strep-tag II, a twin strep tag comprising two identical or different ones of the mentioned tags.

The two sections of the multimerization tag 18 are connected via a spacer having a length of 10 amino acids and the amino acid sequence of SEQ ID NO: 9.

In other embodiments, the sections may be connected via a spacer having a length of up to 15 amino acids or may be located directly adjacent to each other with respect to the primary structure of the polypeptide 10, which means that the spacer is optional.

The multimerization-tag 18 is linked to the C-terminal end of the second domain 16 via a first amino acid linker 32. The first amino acid linker 32 has a length of 6 amino acids and the amino acid sequence of SEQ ID NO: 2.

In other embodiments, the first amino acid linker 32 can have a length of 4, 5, or 6 amino acids.

The C-terminal end of the first domain 14 is linked to the N-terminal end of the second domain 16 via a second amino acid linker 34 having a length of 15 amino acids and the amino acid sequence of SEQ ID NO: 3.

In other embodiments, the second amino acid linker 34 can have a length of 12 to 18 amino acids.

### 2. Multimer of the invention

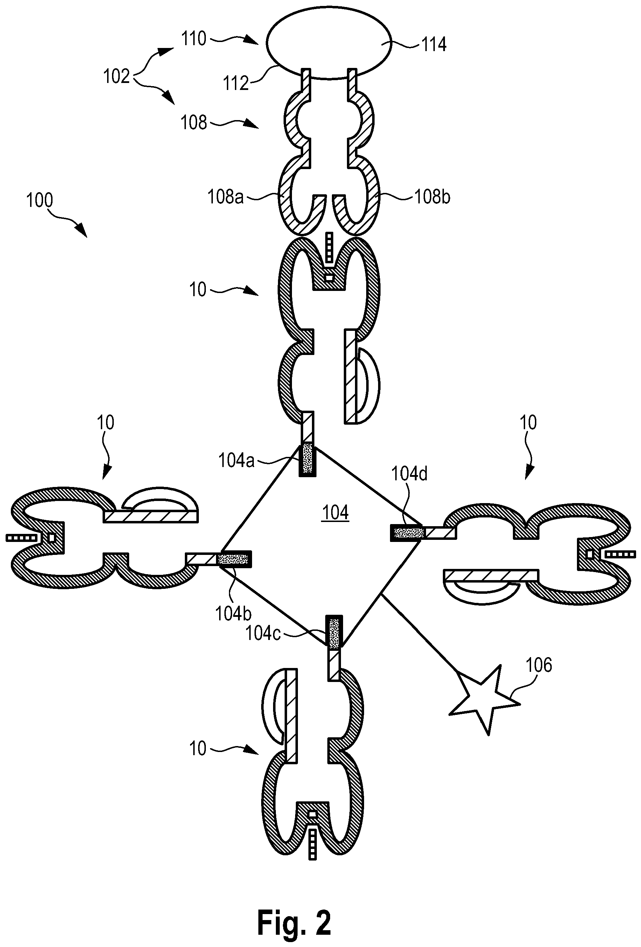

Fig. 2 depicts a schematic view of a multimer 100 according to the invention, four oligopeptides 26, and a target 102 bound to the multimer 100.

The multimer 100 comprises four polypeptides 10 and a streptavidin-derived polyvalent agent 104 bound to the polypeptide 10 via its multimerization-tag 18.

In this embodiment, the multimer 100 is a tetramer comprising four polypeptides 10. However, in other embodiments, the multimer 100 may comprise at least 2 polypeptides 10.

The streptavidin-derived polyvalent agent 104 is streptavidin fused to a signal-residue 106, which is R-phycoerythrin (streptavidin-PE). The streptavidin-PE comprises 4 binding sites 104a, 104b, 104c, and 104d configured to bind a strep-tag II of the multimerization-tag 18. The binding is direct and immediate.

In other embodiments, the streptavidin-derived polyvalent agent may be for example a streptavidin-mutein, a multimer of streptavidin, or a multimer of streptavidin-mutein. Preferably, the polyvalent agent is strep-tactin.

The target 102 is bound to the second region 30 of the second domain 16 of one of the polypeptides 10 constituting the multimer 100. This binding is dependent on the binding of the oligopeptide 26 to the first region 24 of the second domain 16 of the polypeptide 10.

The target 102 comprises a capture molecule 108 and another structure 110. The capture molecule 108 is a TCR specific for the oligopeptide 26 and comprises two amino acid chains 108a and 108b. The other structure 110 is a CD8⁺ T cell comprising a cell-membrane, into which the TCR, i.e., the capture molecule 108, is anchored. The CD8⁺ T cell further comprises a compartment 114 inside, which is its cytoplasm.

In other embodiments, the other structure 110 can be, for example, any cell or a lipid-nanoparticle, preferably a eukaryotic cell, further preferably a T cell.

In other embodiments, the target 102 can be a capture molecule 108 bound to a carrier. The carrier can be, for example, the bottom of a tube, for example plastic, or a material suitable for use as a stationary phase of a chromatographic column or capillary, for example a gel preferably made from dextran or agarose, or a material from which beads or magnetic beads can be made, which are well known in the art.

In all described embodiments, the target 102 can be a capture molecule 108 that is not bound to another structure 110. Furthermore, the capture molecule can be, for example, a protein or a protein-complex comprising fragments of the TCR that mediate the binding of said TCR to the second region 30 of the second domain 16.

### 2. Method for targeting a capture molecule according to the invention

### 2.1 Production of the polypeptide

The polypeptide 10 was produced from a polynucleotide encoding the polypeptide 10 synthesized by GeneCust (Dudelange, Luxembourg) according to the instructions of the inventors. WEHI-231 cells were retrovirally transduced with the polynucleotide, which had been cloned into the expression vector pMXsIP (BioCat, Heidelberg), and subsequently selected using 10 µg/mL puromycin (InvivoGen, Toulouse, France). The supernatants were harvested from transduced cells grown to high density in DMEM containing 10% fetal calf serum (Thermo Fisher, Karlsruhe, Germany).

In this embodiment the polynucleotide further encodes a signal-peptide directly adjacent to the N-terminal end 22 of the polypeptide 10 having the amino acid sequence of SEQ ID NO: 10. This signal-peptide is cleaved apart during production of the polypeptide 10.

In other embodiments the signal-peptide may be another suitable one. Choosing an appropriate leader-peptide for production of a polypeptide 10 in eukaryotic cells is a standard procedure and well known to the skilled person.

In other embodiments, J558L or CHO cells or human cells can be used for production of the polypeptide 10.

13 mL of the supernatant was concentrated to 200 µl by centrifugation through a Vivaspin filter with cut-off 50 kD (Merck, Darmstadt, Germany), in the absence or presence, respectively, of 200 µg/mL oligopeptide 26. The oligopeptide 26 is an HLA-A*01:01-restricted adenoviral peptide epitope and has been synthesized by GeneCust (Dudelange, Luxembourg) according to the instructions of the inventors. During this procedure, the desired oligopeptide 26 was contacted with the polypeptide 10 to generate an oligopeptide-bound polypeptide 10. 100 µl of the concentrated supernatants were incubated with 50 µl streptavidin-beads for 16 h for purification of the polypeptide 10. Afterwards the beads were washed. At this point the polypeptide 10 was removed from the beads according to standard procedures known in the art to obtain the polypeptide 10.

In other embodiments, the supernatant can be concentrated, stored, and be contacted with a desired oligopeptide 26 later, for example by a user.

In all embodiments, the filter used for concentration of the polypeptide from the supernatant can have a cut-off of 50 kD or less than 50 kD.

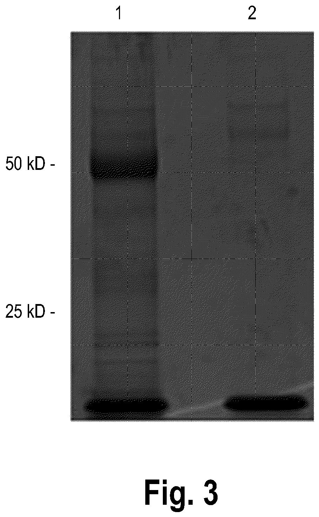

Fig. 3 depicts a photo of an Coomassie-stained reducing SDS-PAGE analysis of the produced polypeptide 10, which was performed to assess expression and correct folding of the polypeptide 10. To the left lane 1 the polypeptide 10 was applied. To the right lane 2 medium was applied as negative control. As visible, the polypeptide 10 was produced as the major product of the procedure. No bands indicating considerable generation of side products or fragmented proteins are visible. Thus, the polypeptide 10 is easily and stably to produce.

### 2.2 Assembly of multimer

Multimerization was conducted by addition of 1 µl streptavidin-PE 104 (Becton Dickinson, Heidelberg, Germany) and 5 µl anti-PE microbeads (Miltenyi-Biotech, Bergisch Gladbach, Germany) for 1 h to 100 µl of concentrated supernatant from WEHI-231 transductants. During this procedure the oligopeptide-bound polypeptide 10 was contacted with the polyvalent agent 104 to generate the multimer 100.

In this embodiment, the streptavidin-derived polyvalent agent is streptavidin-PE, which is multimerized by anti-PE microbeads. This advantageously allows to build the multimer 100 by using streptavidin, which has a limited affinity towards the twin-strep-tag of polypeptide 10 of this embodiment. To build a multimer 100 from polypeptide 10 with solely streptavidin itself as polyvalent agent 104 would not be highly efficient.

In other preferred embodiments, the multimerization tag 18 contains a strep-tag II, more preferably a twin-strep-tag comprising two strep-tag II, and the polyvalent agent 104 is strep-tactin or strep-tactin bound to a signal residue 106.

### 2.3 Targeting the capture molecule

A TCR specific for the oligopeptide 26 is known to the skilled person from literature and was used as capture molecule 108. Polynucleotides encoding a TCRβ 108b chain of said TCR linked to a TCRα chain 108a of said TCR were cloned into retroviral vectors (pMXsIP, BioCat, Heidelberg, Germany) and expressed in TCR-deficient CD8⁺ Jurkat76 T cells. In the polynucleotide, both TCR chain open reading frames were linked using a T2A site and modified as to encode additional cysteine-residues for disulphide bond-mediated stabilization of the TCR.

As a control, a TCR specific for a control-oligopeptide was used. The control-oligopeptide is an HLA-A*03:01-restricted adenoviral peptide epitope. Any other TCR not specific for the oligopeptide 26 may had been used alternatively.

The streptavidin-PE-labelled multimer 100 was used to stain CD8⁺ Jurkat76 T cells expressing either the specific TCR or the control-TCR as negative control. The T cell lines were stained with a solution of multimer 100 for 1 h prior to addition of anti-CD8-FITC and anti-CD3-APC antibodies (BioLegend, Amsterdam, Netherlands) for 30 min. During this procedure, the capture molecule 108 was contacted with the multimer 100 to obtain a targeted capture molecule 108.

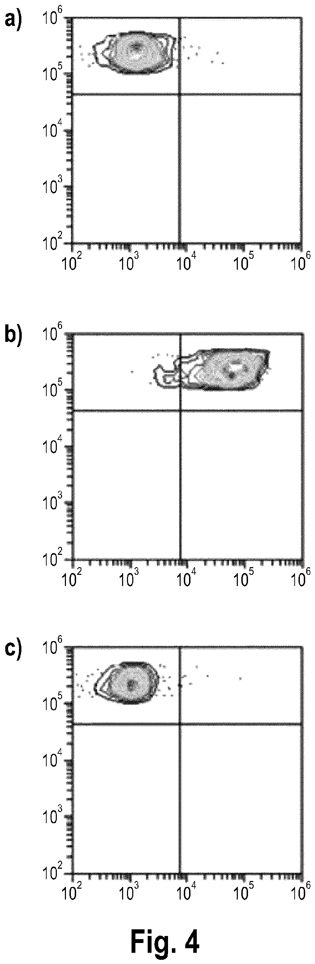

Fig. 4 depicts FACS-plots showing T cells gated on forward and sideward scatter as well as CD3 and CD8 obtained on a CytoFLEX flow cytometer using CytExpert software (Beckman Coulter, Krefeld, Germany). In all three plots on the x-axis there is shown relative fluorescence intensity of PE, which is enhanced the more multimer is bound to the cells. On the x-axis there is shown relative fluorescence intensity of FITC, which is enhanced the more CD8-FITC is bound to the cells.

Fig. 4a relates to Jurkat76 T cells expressing the TCR specific for the oligopeptide 26, which have been stained with the multimer 100 which was not loaded with an oligopeptide. Fig. 4b relates to Jurkat76 T cells expressing the TCR specific for the oligopeptide 26, which have been stained with the multimer 100 which was loaded with the oligopeptide 26. Fig. 4c relates to Jurkat76 T cells expressing the TCR specific for the control-oligopeptide, which have been stained with the multimer 100 which was loaded with the oligopeptide 26.

Unloaded multimer 100 did not stain the T cells expressing the specific TCR (Fig. 4a). By contrast, loaded multimer 100 stained the T cells expressing the specific TCR (Fig. 4b), but not the control T cells (Fig. 4c). The polypeptide 10 is therefore oligopeptide-receptive, can be multimerized by a streptavidin-derived polyvalent agent 104 and can be used for targeting, as, for example, staining and subsequent detection and quantification, of T cells expressing the specific TCR of a bound oligopeptide 26.

### 3. Conclusion

The inventors provide an easy-to-produce and easy-to-multimerize MHC-derived polypeptide with high specificity and controllable avidity towards its capture molecule.

## Claims

1. A polypeptide (10) comprising a single continuous amino acid chain (12), said amino acid chain comprising a first domain (14) derived from β-2-microglobulin, a second domain (16) derived from an MHC-class I α-chain, and a multimerization-tag (18), wherein
- said second domain comprises:
- a first region (24) configured to bind an oligopeptide (26), said first region (24) comprising two α-helices being connected via a disulphide bond (28),
- a second region (30) configured to bind a capture molecule (108) when the first region (24) is oligopeptide-bound; and
- said multimerization-tag (18) being configured to bind a streptavidin-derived polyvalent agent (104).

2. The polypeptide (10) of claim 1, wherein the multimerization-tag (18) is configured to bind reversibly to a streptavidin-derived polyvalent agent (104), such that said binding can be released by elution at 4 °C.

3. The polypeptide (10) of any of the preceding claims, wherein the multimerization-tag (18) comprises at least two identical or different sections, wherein each of said sections is configured to bind a streptavidin-derived polyvalent agent (104).

4. The polypeptide (10) of any of the preceding claims, wherein the multimerization-tag (18) is a twin-strep-tag, preferably said twin strep tag comprising the amino acid sequence of SEQ ID NO: 1.

5. The polypeptide (10) of any of the preceding claims, wherein the multimerization-tag (18) is linked to the C-terminal end of the second domain (16) via a first amino acid linker (32), wherein preferably the first amino acid linker (32) has a length of 4, 5, or 6 amino acids, most preferably the first amino acid linker (32) comprising the amino acid sequence of SEQ ID NO: 2.

6. The polypeptide (10) of any of the preceding claims, wherein the C-terminal end of the first domain (14) is linked to the N-terminal end of the second domain (16) via a second amino acid linker (34), wherein preferably the second amino acid linker (34) has a length of 12 to 18 amino acids, most preferably the second amino acid linker (34) comprising the amino acid sequence of SEQ ID NO: 3.

7. The polypeptide (10) of any of the preceding claims, wherein the capture molecule (108) comprises a T-cell-receptor (TCR), a TCR-like chimeric antigen receptor (TCR-like CAR), or a fragment thereof.

8. The polypeptide (10) of any of the preceding claims, wherein the first domain (14) is derived from a human β-2-microblobuline and preferably comprises the amino acid sequence of SEQ ID NO: 4.

9. The polypeptide (10) of any of the preceding claims, wherein the MHC-class I α-chain is a human leukocyte antigen (HLA), preferably an HLA-A*01.

10. The polypeptide (10) of any of the preceding claims, wherein the amino acid sequence of the polypeptide (10) comprises the amino acid sequence of SEQ ID NO: 5.

11. A kit for targeting a capture molecule (108), said kit comprising the following components:
- a polypeptide (10) according to any of claim 1 to 10 and
- a streptavidin-derived polyvalent agent (104).

12. A polynucleotide encoding the polypeptide (10) of any of claims 1 to 10.

13. A host cell comprising the polynucleotide of claim 12.

14. A multimer (100) comprising:
- the polypeptide (10) of any of claims 1 to 10 and
- a streptavidin-derived polyvalent agent (104) bound to the polypeptide (10) via its multimerization-tag (18).

15. A method for targeting a capture molecule (108) comprising the steps of:
- providing a polypeptide (10) of any of claims 1 to 10;
- contacting a desired oligopeptide (26) with the polypeptide (10) to generate an oligopeptide-bound polypeptide (10);
- contacting the oligopeptide-bound polypeptide (10) with the polyvalent agent (104) to generate a multimer (100); and
- contacting a capture molecule (108) with the multimer (100) to obtain a targeted capture molecule (108).
